# EUROPEAN PATENT APPLICATION

(11) **EP 4 134 052 A1**
(43) Date of publication of application: **15.02.2023**
(21) Application number: 21814177.8
(22) Date of filing: 19.05.2021
(51) Int. Cl.: A61F 13/15, A61F 13/49, A61F 13/496, A61F 13/51

(54) **UNDERPANTS-TYPE ABSORBENT ARTICLE**

(30) Priority: 28.05.2020 JP 2020093622
(71) Applicant: Unicharm Corporation, Ehime 799-0111 (JP)
(72) Inventor: MUKAI, Hirotomo, Kanonji-shi, Kagawa 769-1602 (JP)
(74) Representative: Dolleymores
(86) International application number: PCT/JP2021/018951
(87) International publication number: WO 2021/241349

(57) **Abstract**

An underpants-type absorbent article (1) with both end parts in the transverse direction bonded by a bonding part (50) along the vertical direction, the underpants-type absorbent article (1) being characterized in that: the size (S) of each of a first fused part (51a) and a second fused part (51b) is smaller than the distance (P1) in the vertical direction between the center of the first fused part (51a) and the center of the second fused part (51b); the thickness (11d) of a waist elastic member (11) is smaller than the distance (P2) in the vertical direction between the lower edge of the first fused part (51a) and the upper edge of the second fused part (51b); and the waist elastic member (11) overlaps neither the first fused part (51a) nor the second fused part (51b) in the bonding part (50) when viewed in the front- back direction.

## Description

### FIELD

The present invention relates to an underpants-shaped absorbent article.

### BACKGROUND

There has been known an underpants-shaped absorbent article which includes a liquid-absorbent absorbent main body and an exterior body containing thermoplastic fibers, the exterior body having waist elastic members which stretches and contracts in a lateral direction, two lateral end portions of the exterior body being joined by a joining portion which extends along a vertical direction (e.g., refer to Patent Literature 1).

### [CITATION LIST]

### [PATENT LITERATURE]

Patent Literature 1: Japanese Patent Application Publication No. 2013-126527

### SUMMARY

### [TECHNICAL PROBLEM]

When providing the joining portion in the two lateral end portions of the exterior body and forming the absorbent article into an underpants-shaped state, if a member having a melting point different from that of the exterior body (such as a waist elastic member) is positioned in the fused portion of the joining portion, there is a risk that the exterior body and the waist elastic member or the like are fused and joined together in the fused portion. Then, fusing and joining together the exterior body and the waist elastic member or the like decreases the joining strength of the joining portion, making the joining portion hard. Thus, there is a problem that the wearer is more likely to feel discomfort during usage of the absorbent article.

Further, even in the case where the exterior body is not fused and joined together with the waist elastic member and the like, that the waist elastic member remains in the joining portion makes the joining portion hard. Thus, there is a problem that the wearer is more likely to feel discomfort during usage of the absorbent article.

The present invention was achieved in light of problems such as that described above, and an aspect of the present invention is to provide an underpants-shaped absorbent article in which discomfort during usage of the absorbent article is suppressed by suppressing decrease in the joining strength of a joining portion and hardening of the joining portion.

A main aspect of the present invention for achieving the above-described aspect is an underpants-shaped absorbent article having a vertical direction, a lateral direction, and a front-back direction that intersect each other, the absorbent article including: a liquid-absorbent absorbent main body; and a front waist member and a back waist member that contain thermoplastic fibers, each of the front waist member and the back waist member having a plurality of waist elastic members that stretch and contract in the lateral direction, the front waist member and the back waist member being joined in two lateral end portions and by a joining portion extending along the vertical direction, the joining portion having a first fused portion and a second fused portion, the second fused portion being located below and adjacent to the first fused portion in the vertical direction with a predetermined space, a size of each of the first fused portion and the second fused portion being smaller than a vertical distance between a center of the first fused portion and a center of the second fused portion in a stretched state, a thickness of each of the waist elastic members in a natural state being smaller than a vertical space between a lower end of the first fused portion and an upper end of the second fused portion in the stretched state, in the joining portion, the first fused portion and the second fused portion not overlapping with the waist elastic member when viewed in the front-back direction.

Features of the present invention other than the above will become clear by reading the description of the present specification with reference to the accompanying drawings.

### [ADVANTAGEOUS EFFECT OF THE INVENTION]

According to the present invention, it is possible to provide an underpants-shaped absorbent article in which discomfort during usage of the absorbent article is suppressed by suppressing decrease in the joining strength of a joining portion and hardening of the joining portion.

### BRIEF DESCRIPTION OF DRAWINGS

FIG. 1 is a schematic perspective view of a diaper 1 when viewed from the front side.
FIG. 2 is a schematic plan view and a schematic cross-sectional view of the diaper 1 in an unfolded and stretched state.
FIG. 3 is an enlarged view of a lateral end portion of the diaper 1.
FIG. 4 is diagrams illustrating manufacturing flow from formation of the joining portions 50 until dividing of the continuous diapers 1 into individual diapers 1.
FIG. 5 is diagrams illustrating the difference in the joining state of fused portions 51 depending on the size of a gap space P2 in a state where the fused portion 51 and the waist elastic member 11 overlap.
FIG. 6 is an enlarged view of the lateral end portions of the continuous diapers 1 before dividing into individual diapers, and shows the vertical positions of the fused portions 51 and the waist elastic members 11.
FIG. 7 is a schematic side view of a cut-end portion ce.
FIG. 8 is diagrams illustrating a diamond-shaped fused portion 52, an elongated fused portion 53, and a semicircular fused portion 54.
FIG. 9 is a diagram illustrating that the semicircular fused portions 51c form joining portions by being arranged side-by-side in two rows in the vertical direction.

### DESCRIPTION OF EMBODIMENTS

At least following matters will become clear with description of this specification and attached drawings.

An underpants-shaped absorbent article having a vertical direction, a lateral direction, and a front-back direction that intersect each other, the absorbent article including: a liquid-absorbent absorbent main body; and a front waist member and a back waist member that contain thermoplastic fibers, each of the front waist member and the back waist member having a plurality of waist elastic members that stretch and contract in the lateral direction, the front waist member and the back waist member being joined in two lateral end portions and by a joining portion extending along the vertical direction, the joining portion having a first fused portion and a second fused portion, the second fused portion being located below and adjacent to the first fused portion in the vertical direction with a predetermined space, a size of each of the first fused portion and the second fused portion being smaller than a vertical distance between a center of the first fused portion and a center of the second fused portion in a stretched state, a thickness of each of the waist elastic members in a natural state being smaller than a vertical space between a lower end of the first fused portion and an upper end of the second fused portion in the stretched state, in the joining portion, the first fused portion and the second fused portion not overlapping with the waist elastic member when viewed in the front-back direction.

According to the underpants-shaped absorbent article, the thickness of the waist elastic member in a natural state is smaller than the gap between the fused portions in the vertical direction, and this suppresses the phenomenon that the waist elastic member becomes clogged in the gap at the time of cut back. As a result, it is possible to suppress hardening of the joining portion. Further, since the fused portion and the waist elastic member do not overlap, it is possible to suppress decrease in the joining strength of the joining portion and hardening of the joining portion. Further, the size of the fused portion is smaller than the vertical distance between the centers of the fused portions, and therefore, compared with the case where the fused portion is large, it is possible to reduce the region of the fused portion that becomes hard, and to softly finish the joining portion. That is, it is possible to provide an underpants-shaped absorbent article in which discomfort while putting on the absorbent article is suppressed by suppressing decrease in the joining strength of the joining portion and hardening of the joining portion.

In the underpants-shaped absorbent article, it is desirable that the size of each of the first fused portion and the second fused portion is smaller than the vertical space between the lower end of the first fused portion and the upper end of the second fused portion, in the stretched state.

According to the above underpants-shaped absorbent article, the size of the fused portion is smaller than the vertical space between the fused portions, and therefore, compared with the case where the fused portion is large, it is possible to reduce the region of the fused portion that becomes hard, and to more softly finish the joining portion.

In the underpants-shaped absorbent article, it is desirable that a one end of at least one waist elastic member among the plurality of waist elastic members is positioned inside the joining portion in the lateral direction.

According to the above underpants-shaped absorbent article, it is possible to provide an underpants-shaped absorbent article in which discomfort during usage of the absorbent article is suppressed by suppressing hardening of the joining portion in a portion of the joining portion where the waist elastic member does not exist (a portion where one end is positioned inside).

In the underpants-shaped absorbent article, it is desirable that two ends of all of the plurality of waist elastic members are positioned inside the joining portion in the lateral direction.

According to the above underpants-shaped absorbent article, the waist elastic members do not exist in the joining portion, and this makes it possible to an underpants-shaped absorbent article in which discomfort while putting on the absorbent article is further suppressed by further suppressing hardening of the joining portion.

In the underpants-shaped absorbent article, it is desirable that each of the front waist member and the back waist member has an outer region outside the joining portion in the lateral direction, each of the waist elastic members has a fixed portion that is fixed to either one of the front waist member and the back waist member, and a non-fixed portion that is not fixed, and that, in the stretched state, a farthest outer end of the fixed portion is positioned inside the joining portion in the lateral direction, and a maximum value of a space between an inner end and an outer end of the outer region in the lateral direction is larger than a minimum value of a space between the outer end of the fixed portion and an inner end of the joining portion in the lateral direction.

According to the above underpants-shaped absorbent article, the outer region is more likely to attract attention compared with the case where the lateral space of the outer region is small, and this makes less conspicuous the gap between the outer end of the fixed portion of the waist elastic member and the joining portion, and makes it possible to suppress the deterioration of the appearance. Further, compared with the case where the gap between the outer end of the fixed portion of the waist elastic member and the joining portion is large, the waist elastic member securely contracts (the waist elastic member stretches and contracts within a wide range), and this makes it possible to suppress the slip-down of the underpants-shaped absorbent article.

In the underpants-shaped absorbent article, it is desirable that the maximum value of the space between the inner end and the outer end of the outer region is larger than a value two times the minimum value of the space between the outer end of the fixed portion and the inner end of the joining portion.

According to the above underpants-shaped absorbent article, the outer region are more likely to attracts attention, and this makes less conspicuous the gap between the outer end of the fixed portion of the waist elastic member and the joining portion, making it possible to further suppress the deterioration of the appearance. Further, the underpants-shaped absorbent article more securely contracts, and this makes it possible to further suppress the slip-down of the underpants-shaped absorbent article.

In the underpants-shaped absorbent article, it is desirable that in the stretched state, the size of each of the first fused portion and the second fused portion is 0.5 mm or more and less than 3.0 mm, and the vertical distance between the center of the first fused portion and the center of the second fused portion is 3.0 mm or more and less than 5.0 mm.

According to the above underpants-shaped absorbent article, if the size of the fused portion is less than 0.5 mm, there is a risk of the occurrence of hole generation, not obtaining the required joining strength. If the size of the fused portion is 3.0 mm or more, there is a risk of widening the region which has been hardened by fusion joining, making the wearer feel discomfort while putting on the absorbent article. If the intercenter distance of the fused portions in the vertical direction is less than 3.0 mm, there is a high possibility that the fused portion overlaps the waist elastic member. On the other hand, if the intercenter distance is 5.0 mm or more, the number of the fused portions becomes insufficient, causing a risk that the joining strength is insufficient. That is, it is possible to provide an underpants-shaped absorbent article in which discomfort while putting on the absorbent article is suppressed by obtaining a sufficient joining strength and by suppressing hardening of the joining portion.

In the underpants-shaped absorbent article, it is desirable that each of the front waist member and the back waist member has an adhesive range to which an adhesive for fixing the waist elastic members is applied, and that the joining portion and the adhesive range do not overlap when viewed in the front-back direction.

According to the above underpants-shaped absorbent article, there is a portion where the adhesive is not present exists in the joining portion, and this makes it possible to suppress hardening of the joining portion due to solidification of the adhesive in the portion. Further, if the adhesive is not present in the fused portion, this makes it possible to suppress decrease in the joining strength of the fused portion due to the adhesive at the time of the fusion joining. That is, it is possible to provide an underpants-shaped absorbent article in which discomfort while putting on the absorbent article is suppressed by suppressing decrease in the joining strength of the joining portion and hardening of the joining portion.

In the underpants-shaped absorbent article, it is desirable that each of the front waist member and the back waist member has an adhesive range to which an adhesive for fixing the waist elastic members is applied, that each of the front waist member and the back waist member has an outer region outside the joining portion in the lateral direction, and that the outer region and the adhesive range do not overlap when viewed in the front-back direction.

According to the above underpants-shaped absorbent article, a portion where the adhesive is not present exists in the outer region, and this makes it possible to provide an underpants-shaped absorbent article in which discomfort during usage of the absorbent article is suppressed by suppressing hardening of the portion of the outer region due to solidification of the adhesive.

In the underpants-shaped absorbent article, it is desirable that the first fused portion and the second fused portion are formed by ultrasonic joining.

According to the above underpants-shaped absorbent article, compared with the case of fusion joining by heat sealing, it is possible to rapidly obtain a high joining strength in a short time, and to enhance productivity.

In the underpants-shaped absorbent article, it is desirable that in the underpants-shaped absorbent article that is in the stretched state, within a range of 20 mm downward from an upper end, that the waist elastic members are provided with a predetermined gap in between, in the vertical direction, and that the vertical gap with which the waist elastic members are provided is different from the vertical distance between the center of the first fused portion and the center of the second fused portion.

According to the above underpants-shaped absorbent article, making the gap of the waist elastic members and the intercenter distance of the fused portions different makes it possible to easily arrange the fused portions and the waist elastic members so as not to overlap each other (e.g., the intercenter distance of the fused portions is 3 mm, at positions of 3, 6, 9, 12, 15, and 18 mm from the upper end, and the gap of the waist elastic members is 3.5 mm, at positions of 3.5, 7, 10.5, 14, and 17.5 mm from the upper end).

In the underpants-shaped absorbent article, it is desirable that in the underpants-shaped absorbent article that is in the stretched state, within a range of 20 mm downward from an upper end, the waist elastic members are provided with a predetermined gap in between, in the vertical direction, and either of the following relations is satisfied: the vertical gap with which the waist elastic members are provided is equal to the vertical distance between the center of the first fused portion and the center of the second fused portion, and the vertical gap with which the waist elastic members are provided is equal to or a multiple of the vertical distance between the center of the first fused portion and the center of the second fused portion.

According to the above underpants-shaped absorbent article, by making the gap of the waist elastic members be equal to or a multiple of the intercenter distance of the fused portions, it is possible to easily arrange the fused portions and the waist elastic members so as not to overlap each other (e.g., the fused portions have intercenter distances of 3 mm, from a position at 2 mm away from the upper end, and the waist elastic members have gaps of 3 mm, from a position at 3.5 mm away from the upper end).

In the underpants-shaped absorbent article, it is desirable that an average value of stretch factors of the waist elastic members is equal to or more than 3.0.

According to the above underpants-shaped absorbent article, the waist elastic members are more likely to move back inward in the lateral direction at the time of cut back, compared with a the case where the stretch factor is less than 3.0. This makes it possible to provide an underpants-shaped absorbent article in which discomfort while putting on the absorbent article is suppressed by suppressing hardening of the two lateral end portions of the waist member.

In the underpants-shaped absorbent article, it is desirable that each of the front waist member and the back waist member have a skin-side sheet that is positioned located farthest on a skin side, and a non-skin-side sheet that is located on a non-skin side with respect to the skin-side sheet, and that a total basis weight of the skin-side sheet and the non-skin-side sheet is 32 g/m² or less.

According to the above underpants-shaped absorbent article, compared with the case where the total basis weight is more than 32 g/m², the cost can be reduced. Also, the fused portion does not become excessively hard, and this enables the joining portion to be softly finished and makes it more likely to tear the joining portion when removing the absorbent article. That is, it is possible to provide an underpants-shaped absorbent article whose cost is reduced, which is easy to remove, and in which discomfort while putting on the absorbent article is suppressed.

In the underpants-shaped absorbent article, it is desirable that each of the front waist member and the back waist member has an outer region outside the joining portion in the lateral direction, and that in the stretched state, a maximum value of a space between an inner end and an outer end of the outer region in the lateral direction is larger than a maximum value of a space between an inner end and an outer end of the joining portion in the lateral direction.

According to the above underpants-shaped absorbent article, compared with the case where the lateral space of the outer region is small, it is possible to cut the cut-end portions (lateral outer ends of the waist member (outer region)) at a high line speed (it is possible to divide a continuous underpants-shaped absorbent articles into individual underpants-shaped absorbent articles). Therefore, productivity can be enhanced.

In the underpants-shaped absorbent article, it is desirable that the underpants-shaped absorbent article has a cut-end portion, the cut-end portion being a portion at which a continuous body of the underpants-shaped absorbent articles has been divided into the individual underpants-shaped absorbent articles, the continuous body of the underpants-shaped absorbent articles having a plurality of the underpants-shaped absorbent articles which is in a state of being connected adjacent to each other in the lateral direction, and that the front waist member and the back waist member are at least partially compressed in the cut-end portion.

According to the above underpants-shaped absorbent article, compressing the front and back waist members at the cut-end portions which are provided in the two lateral ends of the waist members makes the two lateral ends of the waist members more likely to be integrated, and therefore it is possible to suppress the deterioration of the appearance compared with the case where the front and back waist members are more likely to separate apart without being compressed.

### Embodiment

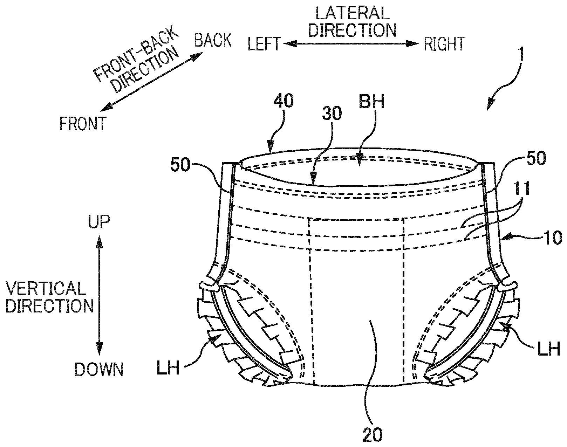

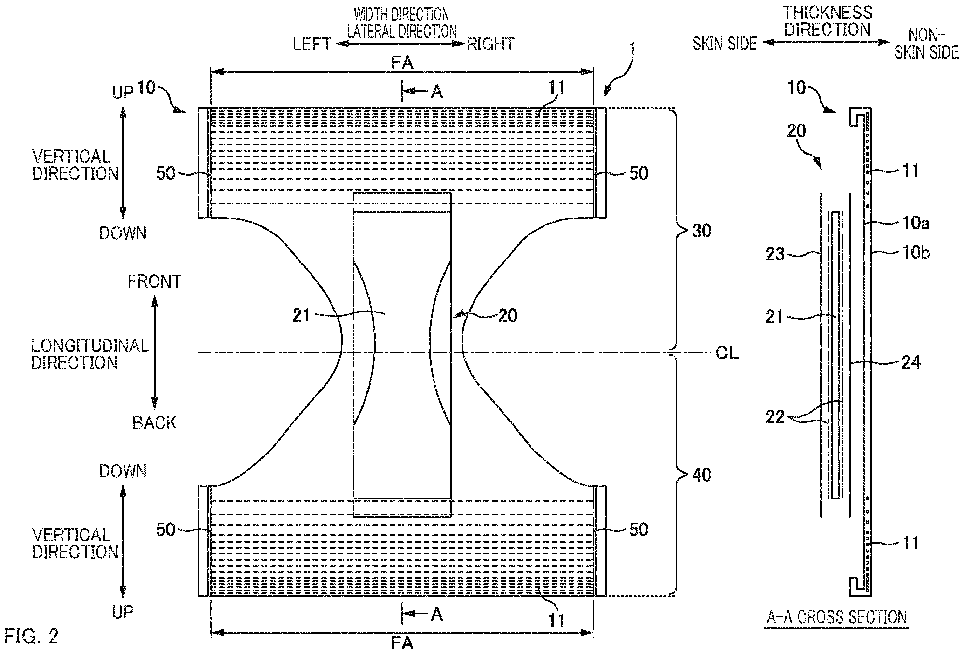

In the present embodiment, as an absorbent article, an underpants-shaped disposable diaper 1 (corresponding to an underpants-shaped absorbent article, hereinafter, also referred to as a "diaper 1") will be described. FIG. 1 is a schematic perspective view of a diaper 1 as viewed from the front side, and FIG. 2 is a plan view of the diaper 1 in an unfolded and stretched state.

It should be noted that, the "stretched state" in FIG. 2 refers to a state where the product (diaper 1) is stretched so as to eliminate wrinkles, specifically, a state where the product (diaper 1) is stretched until the dimensions of constituent members of the diaper 1 (e.g., an exterior body 10, or the like, which will be described later) match or are close to the dimensions of such members on their own. Also, the "natural state" refers to the unstretched state, which is the state in which the elastic bodies contract naturally such that wrinkles or the like appear in the members.

In the underpants-shaped state in FIG. 1, the diaper 1 has three directions that intersect each other, namely a vertical direction, a lateral direction, and a front-back direction. In the following, the one side and the other side in the vertical direction in the underpants-shaped state will also be respectively referred to as "waist side" and "crotch side", and the front side and the back side in the front-back direction will be respectively referred to as the "front side" and the "back side".

On the other hand, in the unfolded and stretched state of FIG. 2, the diaper 1 has three directions that intersect each other, namely a longitudinal direction, a width direction, and a thickness direction. Hereinafter, the one side and the other side in the longitudinal direction in the unfolded state will also be respectively referred to as the "front side" and the "back side". It should be noted that the width direction in the unfolded state is the same direction as the lateral direction in the underpants-shaped state. Therefore, hereinafter, the width direction will also be referred to as the "lateral direction". The longitudinal direction in the unfolded state is a direction along the vertical direction in the underpants-shaped state. Further, in the thickness direction, the side that comes into contact with the skin of the target wearer is defined as the "skin side", and the opposite side thereof is defined as the "non-skin side".

The diaper 1 includes: the exterior body 10; and a liquid-absorbent absorbent main body 20 having a rectangular shape in a plan view and positioned on the skin side of the exterior body 10. As shown in FIGS. 1 and 2, the exterior body 10 includes a portion corresponding to a front waist member 30 and a portion corresponding to a back waist member 40 (hereinafter, "the front waist member 30 and the back waist member 40" will also be referred to as "exterior body 10").

The front waist member 30 is a portion positioned on the wearer's front side while the diaper 1 is put on, and is located on the front side with respect to a longitudinal central position CL in FIG. 2. The back waist member 40 is a portion positioned on the wearer's back side while the diaper 1 is put on, and is located on the back side with respect to the longitudinal central position CL in FIG. 2.

It should be noted that in the diaper 1 of the present embodiment, the front waist member 30 and the back waist member 40 are formed integrally as shown in FIG. 2, but the front waist member 30 and the back waist member 40 may be separate members. For example, a configuration may be acceptable in which a crotch portion (not shown) that is to be located at the wearer's crotch is provided near the longitudinal central position CL between the front waist member 30 and the back waist member 40, and the exterior body 10 is constituted by the front waist member, the back waist member, and the crotch portion. Further, the diaper may have a so-called three-piece configuration (a configuration in which the absorbent main body is arranged so as to span between the front waist member and the back waist member).

The underpants-shaped diaper 1 shown in FIG. 1 is formed in the following manner. First, the exterior body 10 and the absorbent main body 20 in the unfolded state of FIG. 2 are folded one time at a fold position, which is the central position CL in the longitudinal direction (vertical direction). Then, the front waist member 30 and the back waist member 40, which face each other in this folded state, are joined by a pair of joining portions 50 in two lateral end portions by using joining means such as welding. That is, the front waist member 30 and the back waist member 40 are joined by the joining portions 50 that extend along the vertical direction, at two lateral end portions. It should be noted that the lateral end portions of the diaper 1 including the joining portions 50 will be described in detail later.

### Exterior body 10

The exterior body 10 is a portion that constitutes the exterior of the diaper 1, and includes a plurality of flexible sheets (sheet-like members) which contain a thermoplastic resin. The flexible sheets are overlaid on each other in the thickness direction, and are formed of, for example, spunbond nonwoven fabric or the like.

The exterior body 10 according to the present embodiment includes: a skin-side sheet 10a located farthest on the skin side; and a non-skin-side sheet 10b located on the non-skin side with respect to the skin-side sheet. Further, it is preferable that the total basis weight of the skin-side sheet 10a and the non-skin-side sheet 10b is 32 g/m² or less. Therefore, compared with the case where the total basis weight is more than 32 g/m², the cost can be reduced and the joining portion 50 can be softly finished. That is, it is possible to provide the diaper 1 whose cost is reduced and in which discomfort during usage of the diaper is suppressed.

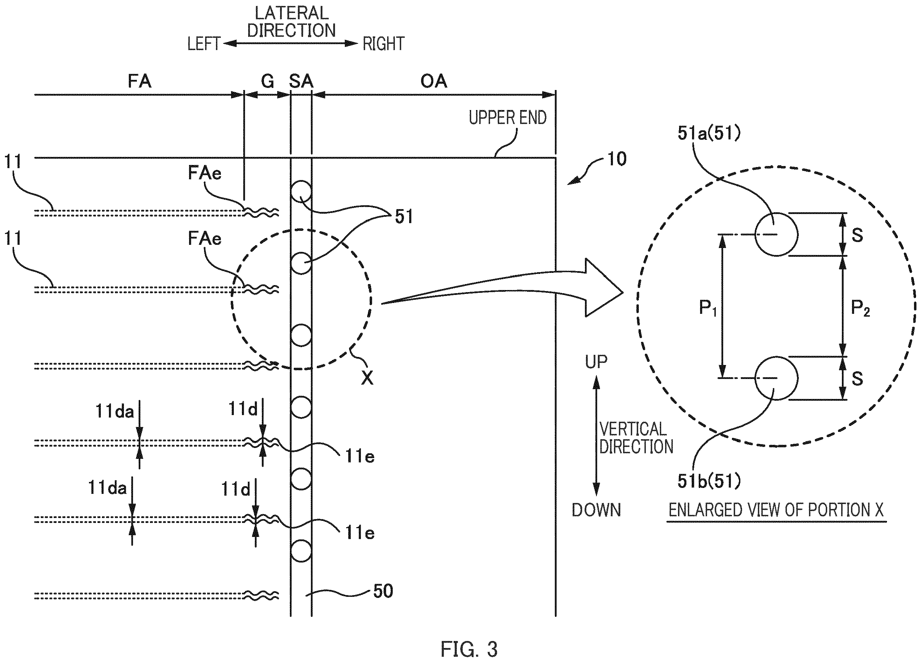

Further, a plurality of waist elastic members 11, each of which stretches and contracts in the lateral direction, are provided between the skin-side sheet 10a and the non-skin-side sheet 10b of the exterior body 10. As the waist elastic member 11, it is possible to use a member having stretchability, such as an elastic string or a stretchable film. It should be noted that, in the present embodiment, elastic strings having a circular cross-sectional shape is used, and the vertical dimension of the waist elastic member 11 in the natural state (corresponding to the thickness of the waist elastic member 11) is represented by 11d, and the vertical dimension of the waist elastic member 11 in the stretched state is represented by 11da (see FIG. 3).

Further, the plurality of waist elastic members 11 are provided being stretched in the exterior body 10, and it is preferable that the average value of the stretch factors of the plurality of waist elastic members 11 is equal to or more than 3.0. Then, compared with the case where the stretch factor is less than 3.0, when the waist elastic members 11 are cut back (a phenomenon that the waist elastic members 11 in the stretched state move back inward in the lateral direction; the detail will be described later), the waist elastic members 11 are more likely to shrink back inward in the lateral direction, and are less likely to remain in the joining portions 50 (in FIG. 3, portions indicated by the wavy shape are each a portion of the waist elastic member 11 which shrink back as a result of cuttting back). This makes it possible to provide the diaper 1 in which discomfort during usage of the diaper is suppressed by suppressing hardening of the joining portions 50. It should be noted that the stretch factor is a value (L1/L0) that indicates the ratio of a total length L1 of the waist elastic member 11 in the stretched state to a total length L0 in the natural no-loaded state (how many times the waist elastic member 11 is stretched when being fixed to the exterior body 10).

Further, the waist elastic members 11 are fixed to the exterior body 10 with adhesive and being stretched within an adhesive range FA shown in FIG. 2. That is, the exterior body 10 has the adhesive range FA to which an adhesive for fixing the waist elastic member 11 is applied. In the present embodiment, it should be noted that the adhesive is directly applied to the waist elastic members 11 within the adhesive range FA, fixing them to the exterior body 10 (the adhesive is not applied to the waist elastic members 11 outside the adhesive range FA). This makes it possible to fix the waist elastic members 11 to the exterior body 10 without applying the adhesive to unnecessary portions.

It should be noted that the adhesive may be applied to each waist elastic member 11 continuously from the left end to the right end within the adhesive range FA. However, in the present embodiment, the adhesive is applied to the waist elastic member 11 intermittently from the left end to the right end within the adhesive range FA. That is, the waist elastic member 11 has a fixed portion that is fixed to the exterior body 10 and a non-fixed portion that is not fixed to the exterior body. Further, the adhesive range FA may be identical or different for all of the waist elastic members 11. For example, the adhesive range FA may be different between the waist elastic members 11 located in a portion that comes into contact with the waist portion and the remaining waist elastic members 11.

### Absorbent main body 20

The absorbent main body 20 has a function of absorbing excrement such as urine, has a substantially rectangular shape in a plan view, and is arranged at the center in the lateral direction, with its longitudinal direction conforming to the vertical direction of the diaper 1 as shown in FIG. 2. The absorbent main body 20 includes: a liquid-absorbent absorbent core 21; a core-wrapping sheet 22 that covers the outer circumferential surfaces of the absorbent core 21; a top sheet 23; and a back film 24.

The absorbent core 21 is obtained by molding liquid-absorbent fibers (e.g., pulp fibers) into a predetermined shape, and the absorbent core has absorbent polymer (so-called SAP) or the like mixed inside thereof.

The core-wrapping sheet 22 is a liquid-permeable sheet member that covers the outer surfaces of the absorbent core 21, and can be constituted by tissue paper, a nonwoven fabric sheet, or the like. It should be noted that the core-wrapping sheet 22 is not necessarily required to be provided.

The top sheet 23 is a liquid-permeable sheet member that is arranged on the skin-side surface of the absorbent core 21 (core-wrapping sheet 22) in the thickness direction, and comes into contact with the wearer's skin while the diaper 1 is put on. The top sheet 23 of the present embodiment is constituted by an air-through nonwoven fabric sheet, a spunbond nonwoven fabric sheet, or the like.

The back film 24 is a liquid-impermeable and moisture-permeable sheet member that is arranged on the non-skin-side surface of the absorbent core 21 (core-wrapping sheet 22) in the thickness direction, and is constituted by a resin film, for example. Providing the back film 24 suppresses transferring (permeation) of moisture such as urine that has been absorbed by the absorbent main body 20, to the wearer's clothes.

### Lateral End Portions of Diaper 1

Next, the lateral end portions of the diaper 1 will be described in detail with reference to the drawings. FIG. 3 is an enlarged view of the lateral end portion of the diaper 1. It should be noted that the end portion of the diaper 1 in the stretched state is described by way of example of the right upper end portion in FIG. 3. However, the present invention is not limited to such a portion. For example, the left upper end portion and the right central end portion can be similarly described.

As shown in FIG. 3, the right upper end portion of the diaper 1 (exterior body 10) includes: the joining portion 50 that is a region extending along the vertical direction and whose lateral dimension SA is indicated; and an outer region that is a region extending along the vertical direction and whose lateral dimension OA is indicated. That is, the exterior body 10 has the outer region outside the joining portion 50 in the lateral direction. It should be noted that in FIGS. 2 and 3, the outer region has a substantially rectangular shape, but the present invention is not limited thereto. For example, a substantially trapezoidal shape whose right side is a short side may be used.

Further, in the diaper 1, the front waist member 30 and the back waist member 40 are fused and joined to each other by the joining portions 50. However, as shown in FIG. 3, the fusing and joining is not made by forming the fused portions continuously in the vertical direction, but the front waist member 30 and the back waist member 40 are fused and joined by the fused portions 51 with a predetermined space.

The enlarged view of the portion X in FIG. 3 shows the fused portions 51 that are adjacent to each other in the vertical direction (the enlarged view of the portion X shows the fused portions 51 located second and third from the upper end; but the present invention is not limited thereto, and, for example, the following description is also applicable to the fused portions 51 located first and second from the upper end). As shown in the enlarged view of the portion X in FIG. 3, of the vertically-adjacent fused portions 51, letting the upper one be a first fused portion 51a, and letting the lower one be a second fused portion 51b, the joining portion 50 has the first fused portion 51a and the second fused portion 51b, the second fused portion 51b being located below and adjacent to the first fused portion 51a in the vertical direction at a predetermined distance (hereinafter, also referred to as a fused-portion distance P1).

Further, it is preferable that this fused-portion distance P1 is 3.0 mm or more and less than 5.0 mm, in the stretched state. If the fused-portion distance P1 is less than 3.0 mm, there is a possibility that the fused portion overlap the waist elastic member 11 (described later in detail), and if the fused-portion distance P1 is 5.0 mm or more, the number of the fused portions 51 becomes insufficient, causing a risk that the joining strength is insufficient. That is, by making the fused-portion distance P1 be 3.0 mm or more and less than 5.0 mm, it is possible to provide the diaper 1 in which discomfort during usage of the diaper is suppressed by preventing the joining strength from being insufficient and by suppressing hardening of the joining portion 50.

In addition, in the present embodiment, when each fused portion 51 is viewed from the thickness direction, the size of a portion having the largest dimension is defined as the size S of the fused portion. The first fused portion 51a and the second fused portion 51b both have a circular shape, and therefore the dimension along any direction conform to the size S of the fused portion. In the enlarged view of the portion X in FIG. 3, the vertical dimension of each of the fused portions is defined as the size S of the fused portion (it should be noted that the case of a shape other than a circular shape will be described later).

Further, it is preferable that the size S of the fused portion is 0.5 mm or more and less than 3.0 mm. If the size S of the fused portion is less than 0.5 mm, there is a risk of the occurrence of hole generation (a phenomenon that through holes are produced in the exterior body 10), not obtaining the required joining strength. If the size of the fused portion is 3.0 mm or more, there is a risk of widening the region which has been hardened by fusion joining, making the wearer feel discomfort during usage of the diaper. That is, if the size S of the fused portion is 0.5 mm or more and less than 3.0 mm, it is possible to provide the diaper 1 in which discomfort during usage of the diaper is suppressed by obtaining a sufficient joining strength and by suppressing hardening of the joining portions 50.

Further, as means for melting the skin-side sheet 10a and the non-skin-side sheet 10b which contain a thermoplastic resin, to form the fused portion 51 in the exterior body 10, it is possible to use an existing formation method such as heat sealing. However, the fused portions 51 (first fused portion 51a and second fused portion 51b) according to the present embodiment are formed by ultrasonic joining. Then, compared with the case of fusion joining by heat sealing, it is possible to rapidly obtain a high joining strength in a short time, and to enhance productivity.

Further, as shown in FIG. 3, the size S of each of the first fused portion 51a and the second fused portion 51b is smaller than the vertical distance between the center of the first fused portion 51a and the center of the second fused portion 51b in the stretched state (fused-portion distance P1). Then, compared with the case where the size S is larger than the fused-portion distance P1, it is possible to make smaller the region occupied by the fused portion 51 in the joining portion 50. This makes it possible to reduce the region of the fused portion 51 that becomes hard, and to softly finish the joining portion 50.

Further, as shown in FIG. 3, the size of each of the first fused portion 51a and the second fused portion 51b is smaller than the vertical space between the lower end of the first fused portion 51a and the upper end of the second fused portion 51b, in the stretched state (hereinafter, also referred to as the gap space P2). Then, compared with the case where the size is larger than the gap space P2, it is possible to make smaller the region occupied by the fused portion 51 in the joining portion 50. This makes it possible to further reduce the region of the fused portion 51 that becomes hard, and to more softly finish the joining portion 50.

Further, as shown in FIG. 3, in the joining portion 50, the first fused portion 51a and the second fused portion 51b do not overlap with the waist elastic members 11 when viewed in the front-back direction. This makes it possible to suppress decrease in the joining strength of the joining portions 50, and to suppress the hardening of the joining portions 50.

More specifically, at the time of making the diaper 1 into an underpants-shaped state by providing the joining portions 50 to two lateral end portions of the exterior body 10 in the diaper 1, if a member having a different melting point and the like from that of the exterior body 10 (e.g., the waist elastic member 11) is positioned in (overlaps with) the fused portion 51 of the joining portion 50, there is a risk that the exterior body 10 and the waist elastic member 11 or the like are fused and joined together in the fused portion 51. Further, if the exterior body 10 and the waist elastic member 11 or the like are fused and joined together, it decreases the joining strength of the joining portion 50, and makes the joining portion 50 hard (e.g., due to the melting and solidification of the waist elastic member 11), making the wearer more likely to feel discomfort during usage of the diaper. In contrast, in the present embodiment, in the joining portions 50, the first fused portion 51a and the second fused portion 51b do not overlap with the waist elastic member 11, and this makes it possible to suppress decrease in the joining strength of the joining portion 50 and hardening of the joining portions 50.

Further, in the present embodiment, the thickness of each waist elastic member 11 in the natural state is smaller than the vertical space (gap space P2) between the lower end of the first fused portion 51a and the upper end of the second fused portion 51b, in the stretched state. This suppresses the phenomenon that the fused portion 51 and the waist elastic member 11 overlap and the phenomenon that the waist elastic member 11 becomes clogged in the gap space P2 at the time of cut back (the phenomenon that the waist elastic member remains in the joining portion 50). As a result, it is possible to suppress decrease in the joining strength of the joining portions 50, and to suppress hardening of the joining portions 50. Hereinafter, such phenomenons will be described in detail with reference to the accompanying drawings.

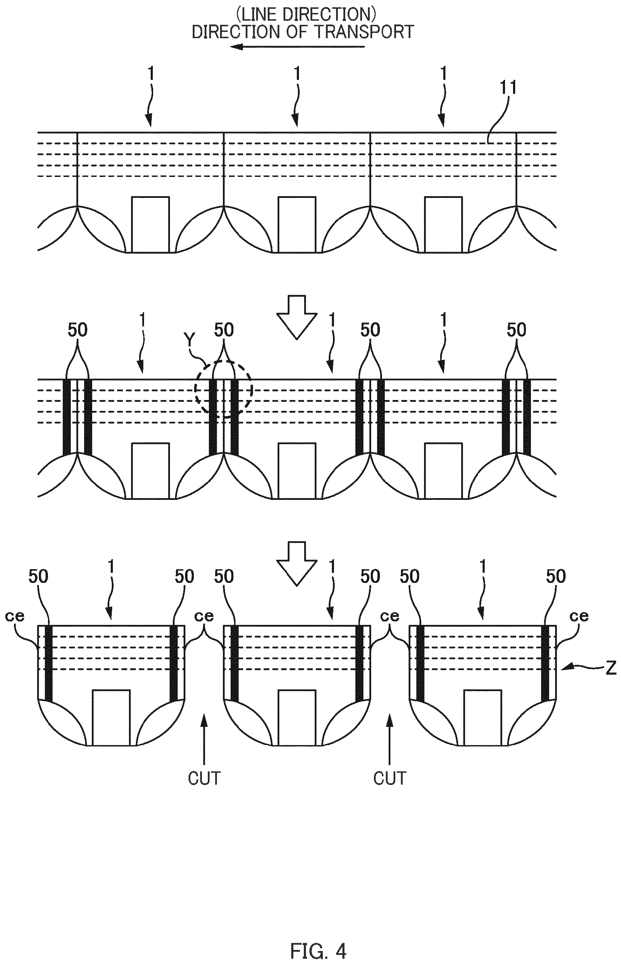

FIG. 4 is diagrams illustrating the manufacturing process of the diaper 1, from formation of the joining portions 50 until dividing of the continuous diapers into individual diapers 1. As shown in FIG. 4, in the manufacturing process of the diaper 1, individual diapers 1 are manufactured while the continuous body of the diapers 1 in which the diapers 1 are connected being adjacent to each other in the lateral direction is transported in the direction of transport (line direction) which is a direction along the lateral direction of the diaper 1. The upper diagram in FIG. 4 shows the diapers 1 before the joining portions 50 are formed, and the diapers 1 that are located continuously in the lateral direction are provided with the waist elastic members 11 that are located continuously in the lateral direction similar thereto.

The center diagram in FIG. 4 shows the diapers 1 after the joining portions 50 are formed. The joining portions 50 are each provided with the plurality of fused portions 51 having the fused-portion distance P1 in the vertical direction, as described above. Further, in order to prevent the fused portion 51 and the waist elastic member 11 from overlapping in advance, it is preferable that constituent members are positioned so as to make not-cut-back waist elastic members 11 pass through the gap space P2 in FIG. 3 (the not-cut-back waist elastic members 11 are ones in a state before formation of the wavy waist elastic members 11 of FIG. 3, and extend to both of the left and right ends as indicated by dashed lines of FIG. 4). However, in some cases, some of the waist elastic member 11 overlap with the fused portion 51 due to positional shift or the like of members.

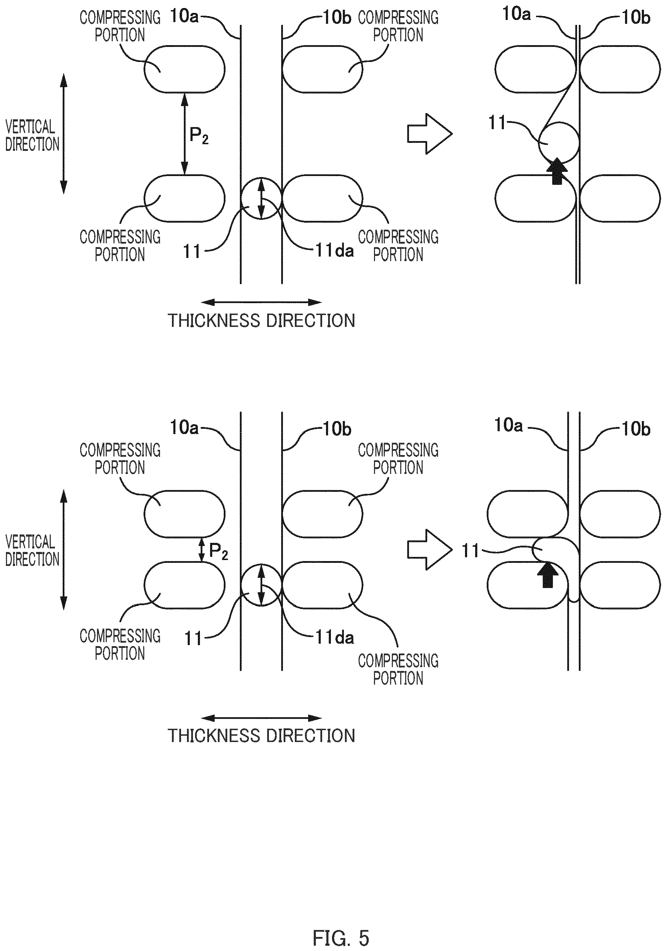

FIG. 5 is diagrams illustrating the difference in the joining state of the fused portions 51 depending on the size of the gap space P2 in a state where the fused portion 51 and the waist elastic member 11 overlap. In the present embodiment, the upper diagram in FIG. 5 shows a case where the gap space P2 is larger than the dimension 11da, and the lower diagram in FIG. 5 shows a case where the gap space P2 is smaller than the dimension 11da. It should be noted that the compressing portion in FIG. 5 is included in an apparatus that forms the fused portion 51, and in the case of ultrasonic joining, one corresponds to an anvil and the other corresponds to a horn.

In the case of the lower diagram in FIG. 5 (if the gap space P2 is smaller than the dimension 11da), when the compressing portion presses the waist elastic member 11, the waist elastic member 11 is moved toward the gap space P2. However, since the gap space P2 is smaller than the dimension 11da, the skin-side sheet 10a and the non-skin-side sheet 10b, and the waist elastic member 11 are fused and joined together. The joining strength is decreased in the joining portion, and the joining portion is hardly finished.

In contrast, in the case of the upper diagram in FIG. 5 (if the gap space P2 is larger than the dimension 11da), when the compressing portion presses the waist elastic member 11, the waist elastic member 11 is moved toward the gap space P2, and is not fused and joined together with the skin-side sheet 10a and the non-skin-side sheet 10b. In this case, only the skin-side sheet 10a and the non-skin-side sheet 10b can be compressed, and fused and joined. That is, it suppresses the phenomenon that the fused portion 51 and the waist elastic member 11 overlap, making it possible to suppress decrease of the joining strength of the joining portion 50 and hardening of the joining portion 50.

It should be noted that, as described above, the dimension 11da is a dimension of the waist elastic member 11 in the stretched state, with respect to the thickness (dimension 11d) of the waist elastic member 11 in the natural state. The dimension 11da can be decreased by decreasing the dimension 11d, for example. That is, that the thickness of the waist elastic member 11 in the natural state is smaller than the gap space P2 makes it possible to suppress the phenomenon shown in the lower diagram in FIG. 5, compared with the case where the thickness of the waist elastic member is large.

The lower diagram in FIG. 4 is a diagram illustrating a state where the continuous diapers 1 has been divided into individual diapers 1. As shown in the lower diagram in FIG. 4, each diaper 1 is cut at a cut-end portion ce. That is, the diaper 1 (exterior body 10) has the cut-end portions ce at which the continuous body of the diapers 1 in which a plurality of diapers 1 are connected being adjacent to each other in the lateral direction, has been divided into individual diapers 1.

Further, when dividing the diapers 1 at the cut-end portions ce, the continuous waist elastic members 11 are also divided into the waist elastic members 11 of the individual diapers 1 at the cut-end portions ce. Further, as described above, the waist elastic members 11 are fixed to the exterior body 10 in the adhesive range FA. That is, as shown in FIG. 3, the waist elastic members 11 positioned outside the lateral outer end FAe of the adhesive range FA contracts up to their natural state (the waist elastic members 11 will be described having wavy shapes for facilitating understanding of the description although the shape is not necessarily a wavy shape). Such a contraction phenomenon of the waist elastic member 11 will be referred to as cut back.

Further, since each waist elastic member 11 immediately after being cut back is in the stretched state, the waist elastic member has the dimension 11da (although smaller than the dimension 11d). But as the waist elastic member contracts, the dimension becomes large and eventually becomes the dimension 11d in the natural state. That is, if the gap space P2 is smaller than the dimension 11d in the natural state, the waist elastic member 11 becomes thick and becomes clogged in the gap space P2 when the waist elastic member 11 contracts due to the cut back, and this causes a risk of not cutting-back the waist elastic member 11 to the state shown in FIG. 3. In the present embodiment, the gap space P2 is larger than the dimension 11d in the natural state, and therefore the generation of such clogging can be suppressed. That is, it is possible to suppress hardening of the joining portion 50 by suppressing the phenomenon that the waist elastic member 11 is clogged in such a gap at the time of cut back (the phenomenon that the waist elastic member remains in the joining portion 50).

Further, as shown in FIG. 3, the outermost end 11e of the waist elastic member 11 which has moved back due to the cut back is positioned inside the joining portion 50 in the lateral direction. That is, among the plurality of waist elastic members 11, the end (outer end 11e) of at least one waist elastic member 11 is positioned inside the joining portion 50 in the lateral direction. Then, it is possible to provide the diaper 1 in which discomfort during usage of the diaper 1 is suppressed by suppressing hardening of the joining portion 50 in a portion of the joining portion 50 where the waist elastic member 11 does not exist (a portion where the outer end 11e is positioned inside).

It should be noted that, in the present embodiment, two ends of all of the plurality of waist elastic members 11 are positioned inside the joining portions 50 in the lateral direction. That is, the waist elastic members 11 do not exist in the joining portions 50, and this makes it possible to provide the diaper 1 in which discomfort during usage of the diaper is further suppressed by further suppressing hardening of the joining portions 50.

Further, as described above, the adhesive for fixing the waist elastic member 11 is applied within the adhesive range FA. That is, when viewed in the front-back direction, the joining portions 50 and the adhesive range FA do not overlap. Then, there is no adhesive in the joining portions 50, and this suppresses hardening of the joining portions 50 due to solidification of the adhesive in the joining portions 50. Further, there is no adhesive in the fused portions 51, and this makes it possible to suppress decrease in the joining strength of the fused portion 51 due to the adhesive at the time of the fusion joining. That is, it is possible to provide the diaper 1 in which discomfort during usage of the diaper is suppressed by suppressing decrease in the joining strength of the joining portions and hardening of the joining portions.

Further, when viewed in the front-back direction, the outer region and the adhesive range FA do not overlap. Accordingly, there is no adhesive in the outer region, and this makes it possible to provide the diaper 1 in which discomfort during usage of the diaper is suppressed by suppressing hardening of the outer region due to solidification of the adhesive.

Further, as shown in FIG. 3, when comparing the maximum value of the lateral dimension OA of the outer region and the maximum value of the lateral dimension SA of the joining portion 50, the maximum value of the dimension OA is larger. That is, in the stretched state, the maximum value (dimension OA) of the space between the inner end and the outer end of the outer region in the lateral direction is larger than the maximum value (dimension SA) of the space between the inner end and the outer end of the joining portion 50 in the lateral direction. Then, compared with the case where the lateral dimension OA of the outer region is small, it is possible to divide the continuous diapers 1 into individual diapers 1 at a high line speed, and this can enhance productivity.

As described above, the diaper 1 is manufactured by dividing a continuous body of the diapers 1 in which the plurality of diapers 1 are connected being adjacent to each other in the lateral direction, into individual diapers 1. As shown in FIG. 4, such dividing into the individual diapers 1 is performed by cutting the connected diapers with aiming at the between-adjacent-joining-portions-50 position which is to be the cut-end portion ce, while transporting the connected diapers 1 in the direction of transport.

That is, at the time of cutting the diapers 1 into individual diapers, it is necessary to cut the cut-end portion ce with an aim. If the dimension OA is smaller than the dimension SA, the tolerance range for the position of the cut-end portion ce in the lateral direction becomes narrow, and this makes it difficult to sufficiently increase the transport rate (line speed). That is, if the dimension OA is larger than the dimension SA as described above, compared with the case where the dimension OA is smaller than the dimension SA, it is possible to cut the continuous body of the diapers 1 at a higher line speed into individual diapers 1, making possible to enhance productivity. It should be noted that as the lateral dimension OA of the outer region increases, the productivity can be further enhanced for the above-described reason. However, as the dimension OA increases, the appearance of the diaper 1 becomes worse. Therefore, it is preferable that the dimension OA is 10 mm or less on average. Making the dimension 10 mm or less on average makes it possible to suppress the deterioration of the appearance of the diaper 1 while enhancing productivity.

Further, as described above, within the adhesive range FA, the adhesive is applied to the waist elastic member 11 intermittently from the left end to the right end, and the waist elastic member 11 has the fixed portion and the non-fixed portion in the adhesive range FA, the fixed portion being a portion that is fixed to the exterior body 10, the non-fixed portion being a portion that is not fixed to the exterior body 10. Further, as shown in FIG. 3, in the stretched state, the farthest outer end of the fixed portion (the outer end FAe of the adhesive range FA) is positioned inside the joining portion 50 in the lateral direction.

Further, when comparing the minimum value of the dimension G from the farthest outer end of the fixed portion to the inner end of the joining portion 50 and the maximum value of the lateral dimension OA of the outer region, the maximum value of the dimension OA is larger. That is, in the stretched state, the maximum value (dimension OA) of the space between the inner end and the outer end of the outer region in the lateral direction is larger than the minimum value (dimension G) of the space between the outer end of the fixed portion and the inner end of the joining portion in the lateral direction. Then, compared with the case where the lateral space of the outer region is small, the outer region is more likely to attract attention. This makes it possible to prevent the gap between the outer end of the fixed portion of each waist elastic member 11 and the joining portion 50 from becoming less conspicuous, and to suppress the deterioration of the appearance. Further, compared with the case where the gap between the outer end of the fixed portion of the waist elastic member 11 and the joining portion 50 is large, the waist elastic member 11 securely contracts (the waist elastic member 11 stretches and contracts within a wide range), and this makes it possible to suppress the slip-down of the diaper 1.

Further, the maximum value (dimension OA) of the space between the inner end and the outer end of the outer region is larger than a value two times the minimum value (dimension G) of the space between the outer end of the fixed portion and the inner end of the joining portion. Then, the outer region are more likely to attract attention, and this makes further less conspicuous the gap between the outer end of the fixed portion of each waist elastic member 11 and the joining portion 50, making it possible to further suppress the deterioration of the appearance. Further, the waist elastic member 11 more securely contracts, and this makes it possible to further suppress the slip-down of the diaper 1. It should be noted that, in order to securely contract the diaper 1, it is preferable that the dimension G is at most 20 mm or less.

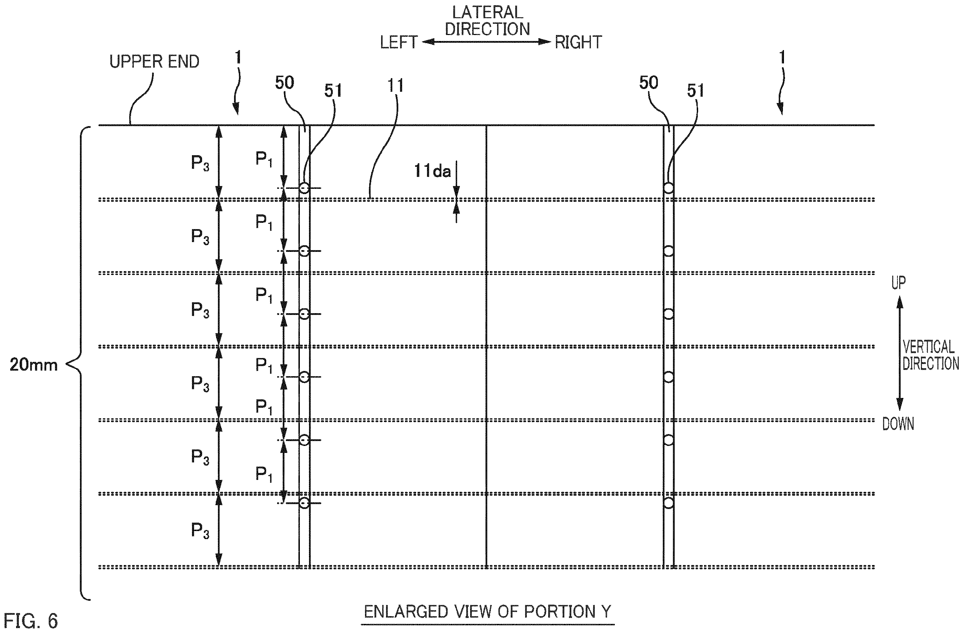

FIG. 6 is an enlarged view of the lateral end portions of the continuous diapers 1 before dividing into individual diapers, illustrating the range Y of FIG. 4, and shows the vertical positions of the fused portions 51 and the waist elastic members 11. It should be noted that, in FIG. 6, there is illustrated a portion that comes mainly into contact with the wearer's waist during usage of the diaper 1, by way of example of a range of 20 mm below from the upper end of the diaper 1 (the portion is not limited to such a range of 20 mm, but corresponds to a so-called waist gather portion).

As shown in FIG. 6, the fused portions 51 are provided with the fused-portion distance P1 in between, in the vertical direction, and the fused-portion distance P1 according to the present embodiment is 3.0 mm. Further, the waist elastic members 11 are provided with a waist-elastic-member gap P3 in the vertical direction, and the waist-elastic-member gap P3 according to the present embodiment is 3.5 mm. That is, in the diaper 1 which is being stretched, within the range of 20 mm downward from its upper end, the waist elastic members 11 are provided with a predetermined gap (waist-elastic-member gap P3) in between, in the vertical direction. In the vertical direction, the gap with which the waist elastic members 11 are provided (waist-elastic-member gap P3) is different from the distance between the center of the first fused portion 51a and the center of the second fused portion 51b (fused-portion distance P1).

This makes it possible to easily arrange the fused portions 51 and the waist elastic members 11 so as not to overlap each other in the thickness direction. For example, in the case of setting the gap and the distance as described above, the fused portions 51 are positioned with a distance of 3 mm, at positions of 3, 6, 9, 12, 15, and 18 mm from the upper end, and the waist elastic members 11 are positioned with a gap of 3.5 mm, at positions of 3.5, 7, 10.5, 14, and 17.5 mm from the upper end. This makes it possible to arrange the fused portions 51 so as not to overlap the waist elastic members 11 in the thickness direction.

Further, in the foregoing description, by making the fused-portion distance P1 and the waist-elastic-member gap P3 different from each other, the fused portions 51 and the waist elastic members 11 are arranged so as not to overlap in the thickness direction. But, the present invention is not limited thereto. For example, a configuration may be acceptable in which, in the vertical direction, the gap with which the waist elastic members 11 are provided (waist-elastic-member gap P3) is equal to the distance between the centers of the first fused portion 51a and the second fused portion 51b (fused-portion distance P1), or in which, in the vertical direction, the gap with which the waist elastic members 11 are provided (waist-elastic-member gap P3) is equal to or a multiple of the distance between the centers of the first fused portion 51a and the second fused portion 51b (fused-portion distance P1).

Then, in such a case, it is possible to easily arrange the fused portions 51 and the waist elastic members 11 so as not to overlap each other. For example, the fused portions 51 are arranged intermittently from the position at 2 mm away from the upper end, with the fused-portion distances P1 of 3 mm, and the waist elastic members 11 are arranged intermittently from the position at 3.5 mm away from the upper end, with the waist-elastic-member gaps P3 of 3.5 mm. This makes it possible to arrange the fused portions 51 and the waist elastic members 11 so as not to overlap.

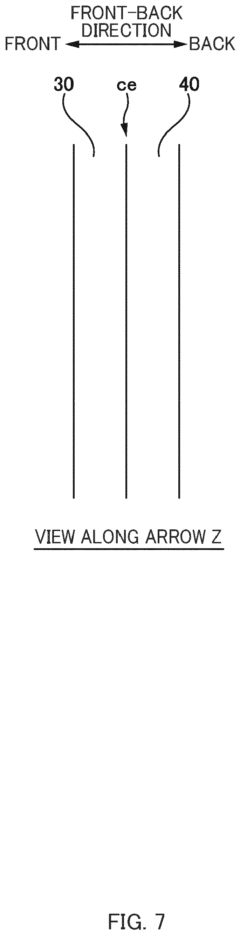

FIG. 7 is a schematic side view of the cut-end portion ce. In the present embodiment, the cutting of the cut-end portion ce is performed by press-cutting the cut-end portion ce using a cutting tool, and therefore the cut-end portions can be in a state of being in close contact with each other and compressed as shown in FIG. 7. With reference to FIG. 4, at the timing of changing the state shown in the center diagram in FIG. 4 to the state shown in the lower diagram in FIG. 4, the cut-end portion ce is compressed and cut by pressing a cutting tool provided on the front side of the continuous body of the diapers 1 (the near side in the direction of penetrating the paper surface in FIG. 4) against a receiving blade portion provided on the back side of the continuous body of the diapers 1 (the far side in the direction of penetrating the paper surface in FIG. 4). Then, the front waist member 30 and the back waist member 40 are considerably compressed in the front-back direction by the cutting tool and the receiving blade portion, thus forming a compressed portion. That is, in the cut-end portion ce, the front waist member 30 and the back waist member are at least partially compressed. This makes two lateral ends of the exterior body 10 more likely to be integrated, and therefore it is possible to suppress the deterioration of the appearance compared with the case where the waist members have separated apart.

### Other Embodiments

The above embodiment has been described in order to facilitate the understanding of the present invention, not to interpret the present invention in a limited manner. The present disclosure may variously be changed or altered without departing from its gist and encompass equivalents thereof.

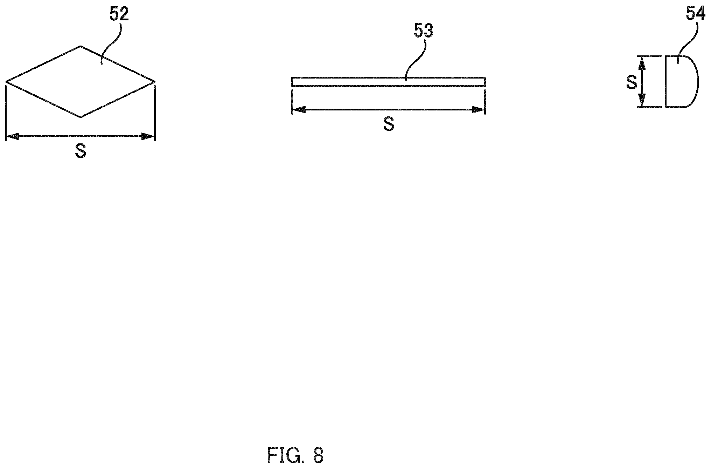

Further, in the above embodiment, the fused portions 51 each has a circular shape, but the present invention is not limited thereto. FIG. 8 is diagrams illustrating a diamond-shaped fused portion 52, an elongated fused portion 53, and a semicircular fused portion 54. That is, the fused portion may be one having a shape other than a circular shape as shown in FIG. 8. It should be noted that, the size S of the fused portion is defined, as described above, as the size of a portion having a largest dimension when viewed from the thickness direction. Therefore, the size S of each of the fused portions depends on their shapes, and, in FIG. 8, the sizes S of the fused portions are as shown in the shapes.

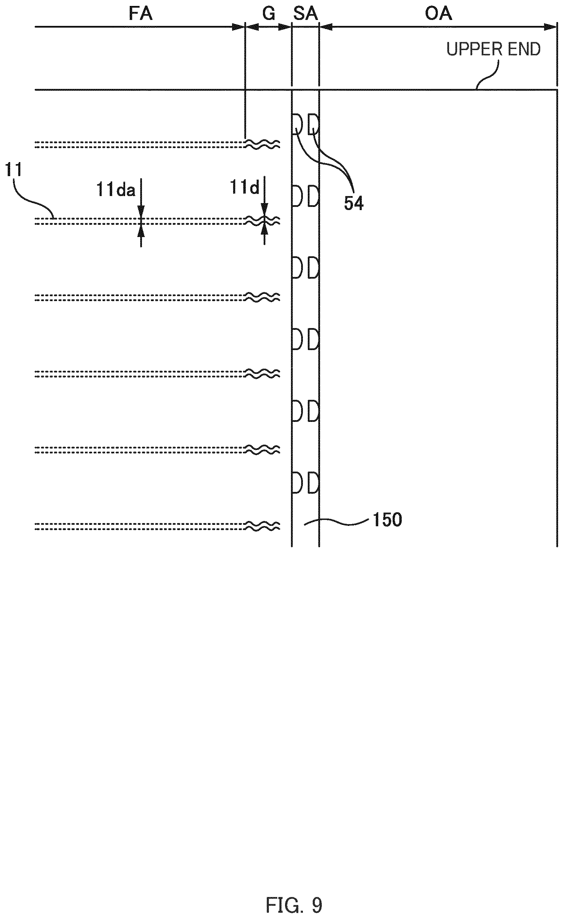

Further, in the above embodiment, the joining portions 50 are formed by arranging the fused portions 51 side-by-side in one row in the vertical direction. However, the present invention is not limited thereto, and the joining portions may be formed by a plurality of rows of the fused portions. FIG. 9 is a diagram corresponding to FIG. 3, and in which the semicircular fused portions 54 form joining portions 150 by being arranged side-by-side in two rows in the vertical direction. In FIG. 9, the lateral dimension SA of the joining portion 150 is the dimension SA from the left end of the left semicircular fused portion 54 to the right end of the right semicircular fused portion 54. Then, compared with the case where the fused portions are arranged side-by-side in one row in the vertical direction, the dimension SA becomes larger and the fused region of the joining portion 150 becomes wider, making it possible to make the joining secure. Compared with the case where the fused portions are continuously formed in the lateral direction of the dimension SA, the fused region of the fused portion becomes smaller (there is an unfused region at the center). This makes it possible to finish the joining portion 150 softly and makes it easier to tear it when removing the diaper (it makes easier to tear the joining portions, by tearing one or both of the joining portions of the diaper, at the time of unfolding the diaper from an underpants shape into a shape in which the diaper is easy to remove from the wearer).

Further, regarding the arrangement of the fused portions in the joining portion, the joining portion can be formed in a state where the elongated fused portions 53 are tilted with respect to the lateral direction (in a tilted state) (it is possible to form fused portions that are oriented obliquely upward-right, from the left end to the right end of the joining portion), for example. Thus, having a predetermined angle with respect to the direction of transport, makes it possible to avoid the use of a specific place of the horn, and to use the entirety of the horn. This can reduce the necessity (frequency) of maintenance for polishing due to wear, and the time required for maintenance and parts replacement.

More specifically, in the case of ultrasonic sealing, the continuous body of the diapers 1 shown in the upper diagram in FIG. 4 is transported so as to pass between the anvil roll and the ultrasonic horn, and the interaction between the anvil-roll surface pattern and the corresponding ultrasonic horn forms a portion of the finished product which functions as the joining portion. For example, the anvil roll rotates, and has a plurality of pattern blocks composed of a pattern of two joining portions, as shown in the center diagram in FIG. 4, namely the one-side portion of the two lateral side portions of a product in which the front waist member 30 and the back waist member 40 are in the folded state, and the other-side portion of the two lateral side portions of the immediately adjacent product. Each pattern block corresponds to an identical ultrasonic horn. At this time, as shown in the lower diagram in FIG. 4, two patterns are arranged next to the line of the cut-end portion ce in the direction of transport. However, arranging the patterns symmetrically with respect to the line of the cut-end portion ce can reduce the frequency of contacting the same location. If the angle of the pattern is excessively large, the joining portions are more likely to overlap the elastic members, and therefore it is recommended that the tilted angle is made as small as possible.

Regarding the arrangement of the fused portions in the joining portion, it is possible to use the combination of the fused portion 51, the diamond-shaped fused portion 52, the elongated fused portion 53, and the semicircular fused portion 54. For example, the joining portion can be formed by the following fused portions: the fused portion 51 is used in a portion that comes into contact with the waist portion; the diamond-shaped fused portion 52 is used below the foregoing portion; and the semicircular fused portions 54 shown in FIG. 9 are arranged therebelow in two rows in the vertical direction. Then, the joining strength, appearance, ease of tearing at the time of removal, and the like can be adjusted depending on the position of the joining portion in the vertical direction.

Further, in the above embodiment, only the waist elastic members 11 are provided, but the present invention is not limited thereto. For example, a configuration is acceptable in which a portion that comes into contact with the waist portion has the waist elastic members 11, and the remaining portions has elastic strings or stretchable films having a substantially rectangular cross-sectional shape.

Further, in the above embodiment, all of the waist elastic members 11 are cut back through from the upper end to the lower end of the joining portion 50, but the present invention is not limited thereto. For example, even when waist elastic members 11 provided in a portion that comes into contact with the waist portion are cut back and waist elastic members 11 provided in the remaining portions are not cut back, the present invention can be applied to the cut-back waist elastic members 11.

### REFERENCE SIGNS LIST

1: diaper (underpants-type absorbent article),
10: exterior body, 10a: skin-side sheet, 10b: non-skin-side sheet,
11: waist elastic member, 11d: dimension, 11da: dimension, 11e: outer end,
20: absorbent main body, 21: absorbent core, 22: core-wrapping sheet
23: top sheet, 24: back film,
30: front waist member, 40: back waist member,
50: joining portion, 51: fused portion, 51a: first fused portion,
51b: second fused portion,
52: diamond-shaped fused portion, 53: elongated fused portion,
54: semicircular fused portion, 150: joining portion
BH: waist opening, LG: leg opening, CL: central position, FA: adhesive range,
FAe: outer end, OA: dimension, SA: dimension, G: dimension, S: size of fused portion
P1: fused-portion distance,
P2: gap space,
P3: waist-elastic-member gap,
ce: cut-end portion

## Claims

1. An underpants-shaped absorbent article having a vertical direction, a lateral direction, and a front-back direction that intersect each other,
the absorbent article comprising:
a liquid-absorbent absorbent main body; and
a front waist member and a back waist member that contain thermoplastic fibers,
each of the front waist member and the back waist member having a plurality of waist elastic members that stretch and contract in the lateral direction,
the front waist member and the back waist member being joined in two lateral end portions and by a joining portion extending along the vertical direction,
the joining portion having a first fused portion and a second fused portion,
the second fused portion being located below and adjacent to the first fused portion in the vertical direction with a predetermined space,
a size of each of the first fused portion and the second fused portion being smaller than a vertical distance between a center of the first fused portion and a center of the second fused portion in a stretched state,
a thickness of each of the waist elastic members in a natural state being smaller than a vertical space between a lower end of the first fused portion and an upper end of the second fused portion in the stretched state,
in the joining portion, the first fused portion and the second fused portion not overlapping with the waist elastic member when viewed in the front-back direction.

2. The underpants-shaped absorbent article according to claim 1, wherein
the size of each of the first fused portion and the second fused portion is smaller than the vertical space between the lower end of the first fused portion and the upper end of the second fused portion, in the stretched state.

3. The underpants-shaped absorbent article according to claim 1 or 2, wherein
a one end of at least one waist elastic member among the plurality of waist elastic members is positioned inside the joining portion in the lateral direction.

4. The underpants-shaped absorbent article according to claim 3, wherein
two ends of all of the plurality of waist elastic members are positioned inside the joining portion in the lateral direction.

5. The underpants-shaped absorbent article according to any one of claims 1 to 4, wherein
each of the front waist member and the back waist member has an outer region outside the joining portion in the lateral direction,
each of the waist elastic members has
a fixed portion that is fixed to either one of the front waist member and the back waist member, and
a non-fixed portion that is not fixed, and
in the stretched state,
a farthest outer end of the fixed portion is positioned inside the joining portion in the lateral direction, and
a maximum value of a space between an inner end and an outer end of the outer region in the lateral direction
is larger than
a minimum value of a space between the outer end of the fixed portion and an inner end of the joining portion in the lateral direction.

6. The underpants-shaped absorbent article according to claim 5, wherein
the maximum value of the space between the inner end and the outer end of the outer region
is larger than
a value two times the minimum value of the space between the outer end of the fixed portion and the inner end of the joining portion.

7. The underpants-shaped absorbent article according to any one of claims 1 to 6, wherein
in the stretched state,
the size of each of the first fused portion and the second fused portion is 0.5 mm or more and less than 3.0 mm, and
the vertical distance between the center of the first fused portion and the center of the second fused portion is 3.0 mm or more and less than 5.0 mm.

8. The underpants-shaped absorbent article according to any one of claims 1 to 7, wherein
each of the front waist member and the back waist member has an adhesive range to which an adhesive for fixing the waist elastic members is applied, and
the joining portion and the adhesive range do not overlap when viewed in the front-back direction.

9. The underpants-shaped absorbent article according to any one of claims 1 to 7, wherein
each of the front waist member and the back waist member has an adhesive range to which an adhesive for fixing the waist elastic members is applied,
each of the front waist member and the back waist member has an outer region outside the joining portion in the lateral direction, and
the outer region and the adhesive range do not overlap when viewed in the front-back direction.

10. The underpants-shaped absorbent article according to any one of claims 1 to 9, wherein
the first fused portion and the second fused portion are formed by ultrasonic joining.

11. The underpants-shaped absorbent article according to any one of claims 1 to 10, wherein
in the underpants-shaped absorbent article that is in the stretched state, within a range of 20 mm downward from an upper end,
the waist elastic members are provided with a predetermined gap in between, in the vertical direction, and
the vertical gap with which the waist elastic members are provided is different from the vertical distance between the center of the first fused portion and the center of the second fused portion.

12. The underpants-shaped absorbent article according to any one of claims 1 to 10, wherein
in the underpants-shaped absorbent article that is in the stretched state, within a range of 20 mm downward from an upper end,
the waist elastic members are provided with a predetermined gap in between, in the vertical direction, and
either of the following relations is satisfied:
the vertical gap with which the waist elastic members are provided is equal to the vertical distance between the center of the first fused portion and the center of the second fused portion, and
the vertical gap with which the waist elastic members are provided is equal to or a multiple of the vertical distance between the center of the first fused portion and the center of the second fused portion.

13. The underpants-shaped absorbent article according to any one of claims 1 to 12, wherein
an average value of stretch factors of the waist elastic members is equal to or more than 3.0.

14. The underpants-shaped absorbent article according to any one of claims 1 to 13, wherein
each of the front waist member and the back waist member have
a skin-side sheet that is positioned located farthest on a skin side, and
a non-skin-side sheet that is located on a non-skin side with respect to the skin-side sheet, and
a total basis weight of the skin-side sheet and the non-skin-side sheet is 32 g/m² or less.

15. The underpants-shaped absorbent article according to any one of claims 1 to 14, wherein
each of the front waist member and the back waist member has an outer region outside the joining portion in the lateral direction, and
in the stretched state,
a maximum value of a space between an inner end and an outer end of the outer region in the lateral direction
is larger than
a maximum value of a space between an inner end and an outer end of the joining portion in the lateral direction.

16. The underpants-shaped absorbent article according to any one of claims 1 to 15, wherein
the underpants-shaped absorbent article has a cut-end portion,
the cut-end portion being a portion at which a continuous body of the underpants-shaped absorbent articles has been divided into the individual underpants-shaped absorbent articles,
the continuous body of the underpants-shaped absorbent articles having a plurality of the underpants-shaped absorbent articles which is in a state of being connected adjacent to each other in the lateral direction, and
the front waist member and the back waist member are at least partially compressed in the cut-end portion.
